# EUROPEAN PATENT APPLICATION

(11) **EP 3 682 883 A1**
(43) Date of publication of application: **22.07.2020**
(21) Application number: 18855594.0
(22) Date of filing: 14.09.2018
(51) Int. Cl.: A61K 31/4709, A61P 35/00, A61P 5/14, A61P 5/00

(54) **QUINOLINE DERIVATIVE FOR TREATMENT OF NEUROENDOCRINE TUMORS**

(30) Priority: 15.09.2017 CN 201710833310
(71) Applicant: Chia Tai Tianqing Pharmaceutical Group Co., Ltd., Lianyungang, Jiangsu 222062 (CN)
(72) Inventor: ZHANG, Xiquan, Lianyungang Jiangsu 222062 (CN); WANG, Xunqiang, Lianyungang Jiangsu 222062 (CN); JIANG, Hai, Lianyungang Jiangsu 222062 (CN); TIAN, Xin, Lianyungang Jiangsu 222062 (CN); YANG, Ling, Lianyungang Jiangsu 222062 (CN)
(74) Representative: Bianchetti Bracco Minoja S.r.l.
(86) International application number: PCT/CN2018/105628
(87) International publication number: WO 2019/052520

(57) **Abstract**

A quinoline derivative 1-[[[4-(4-fluoro-2-methyl-1H-in dol-5-yl)oxy-6-methoxyquinolin-7-yl]oxy]methyl]cyclopropylamine as represented by the following formula (I) for treatment of neuroendocrine tumors. Also disclosed is an application of the quinoline derivative in preparation of pharmaceutical compositions/medicaments for treatment of tumors, particularly neuroendocrine tumors.

## Description

### FIELD OF THE INVENTION

The invention belongs to the field of medicine, and relates to a use of a quinoline derivative in the preparation of a pharmaceutical composition for tumor treatment. In particular, the invention relates to the use of quinoline derivatives in the treatment of neuroendocrine tumors.

### BACKGROUND OF THE INVENTION

Neuroendocrine tumors (NETs) originate from neuroendocrine cells of the diffuse endocrine system throughout the body. The incidence of NETs has increased from 1.09 cases per 100,000 people in 1973 to 5.25 cases per 100,000 people in 2004. More than a third of the patients has undergone locally advanced or metastatic NETs when initially diagnosed. The most common types of NETs include carcinoid tumors (mostly originating in the lung, bronchus, small intestine, appendix, rectum and thymus) and pancreatic neuroendocrine tumors. Other less common types include those of parathyroid, thyroid, adrenal, and pituitary origin. According to the origin of the primary tumor, the most common neuroendocrine tumor is a gastrointestinal pancreatic neuroendocrine tumor.

Most of the NETs are scattered, and the pathogenic factors of scattered NETs are still poorly understood. NETs may also originate from genetic syndromes, including MEN1 (multiple endocrine neooplasia type 1) and MEN2. MEN1 is associated with menin gene mutations and is associated with a variety of tumor types originating from parathyroid, pituitary, pancreas and the like. MEN2 is associated with mutations in the RET gene, and is associated with the development of medullary thyroid carcinoma, pheochromocytoma, hyperparathyroidism, and the like.

Patients with NETs behave differently depending on the secretion of hormones. Symptoms include intermittent flushing and diarrhea in patients with carcinoid syndrome, hypertension in patients with pheochromocytoma, and those arising from secretion of insulin, glucagon, gastric-stimulating fluid as well as other peptides in patients with pancreatic neuroendocrine tumors. Patients with hormonal symptoms are considered to have a functional tumor, and asymptomatic patients are considered to have a nonfunctional tumor.

NETs are classified histologically based on tumor differentiation and tumor grade (grades 1-3). Histologically, it can be classified according to the degree of tumor differentiation (well differentiated, poorly differentiated) and tumor grade (grades 1-3). Most NETs can be classified into three categories: well differentiated, low grade (G1); well differentiated, intermediate grade (G2); poorly differentiated, high grade(G3). Tumor differentiation and tumor grade are often related to mitosis count and Ki-67 proliferation index. Many studies have confirmed that a high mitotic rate and a high Ki-67 index are associated with greater aggressiveness and poor prognosis of NETs.

Radical surgery is the only way to cure NETs. Other systemic treatments for locally advanced or metastatic G1 / G2 neuroendocrine tumors include somatostatin analogues (SSAs), interferon-α, peptide receptor radionuclide therapy(PRRT) combined somatostatin analogues, Targeted therapy and chemotherapy drugs. Cytotoxic chemotherapy drugs are also the main treatment for G3 neuroendocrine tumors. The FDA has approved the molecular targeted drugs sunitinib and everolimus for the treatment of pancreatic neuroendocrine tumors.

### SUMMARY OF THE INVENTION

In one aspect, the application provides a method of treating a neuroendocrine tumor, the method comprises administering a therapeutically effective amount of Compound I or a pharmaceutically acceptable salt thereof to a patient in need of treatment.

The chemical name of compound I is 1-[[[[4-(4-fluoro-2-methyl-1H-indol-5-yl) oxy-6-methoxyquinolin-7-yl]oxy]methyl]cyclopropylamine, which has the following structural formula:

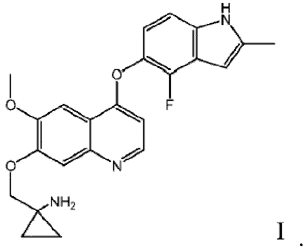

In another aspect, the present application provides Compound I or a pharmaceutically acceptable salt thereof for use in the treatment of a neuroendocrine tumor, or for use in the preparation of a medicament for treating a neuroendocrine tumor.

In yet another aspect, the present application provides a pharmaceutical composition for treating a neuroendocrine tumor, the pharmaceutical composition comprising Compound I or a pharmaceutically acceptable salt thereof, and at least one pharmaceutically acceptable carrier.

In yet another aspect, the present application provides a kit for treating a neuroendocrine tumor, comprising (a) a pharmaceutical composition of at least one unit dose of Compound I or a pharmaceutically acceptable salt thereof, and (b) instructions for treating the neuroendocrine tumor.

In one aspect, the present application provides a method of treating a neuroendocrine tumor, the method comprising administering a therapeutically effective amount of Compound I or a pharmaceutically acceptable salt thereof to a patient in need of treatment.

In some typical embodiments, the neuroendocrine tumor includes, but is not limited to, carcinoid and pancreatic neuroendocrine tumor.

In some typical embodiments, the neuroendocrine tumor includes but is not limited to gastrointestinal neuroendocrine tumor, pulmonary neuroendocrine tumor, pancreatic neuroendocrine tumor, liver neuroendocrine tumor, prostate neuroendocrine tumor, bile duct neuroendocrine tumor, Breast neuroendocrine tumor, ampulla neuroendocrine tumor, thymus neuroendocrine tumor, mediastinal neuroendocrine tumor, retroperitoneal neuroendocrine tumor, thyroid neuroendocrine tumor, and adrenal neuroendocrine tumor.

In some typical embodiments, the neuroendocrine tumor is a poorly differentiated neuroendocrine tumor.

In some typical embodiments, the neuroendocrine tumor is a well differentiated neuroendocrine tumor.

In some typical embodiments, the neuroendocrine tumor is advanced and / or metastatic neuroendocrine tumor.

In some embodiments, the neuroendocrine tumor is advanced and / or metastatic well differentiated neuroendocrine tumor.

In some embodiments, the neuroendocrine tumor is the one after failure of treatment with radiotherapy and / or chemotherapy.

In some embodiments, the neuroendocrine tumor is the one after failure of treatment with a molecular targeted drug (including but not limited to sunitinib or a salt thereof and / or everolimus). In some embodiments, the neuroendocrine tumor is the one after failure of treatment with sunitinib or a salt thereof and / or everolimus.

In some embodiments, the neuroendocrine tumor is the one after failure of treatment with a somatostatin analog and / or an interferon- α and / or peptide receptor radionuclide.

In some embodiments of the present application, a method of treating a pancreatic neuroendocrine tumor is provided, comprising administering a therapeutically effective amount of Compound I or a pharmaceutically acceptable salt thereof to a patient in need of treatment.

In some embodiments, the pancreatic neuroendocrine tumor includes islet cell tumor, insulinoma, amine precursor uptake and decarboxylation cell tumors (APUDomas), vasoactive intestinal peptide tumor (VIP tumor), pancreatic peptide tumor, glucagon Tumor, and gastrin tumor.

In some typical embodiments, the pancreatic neuroendocrine tumor is a poorly differentiated neuroendocrine tumor. In some typical embodiments, the pancreatic neuroendocrine tumor is a well differentiated neuroendocrine tumor.

In some typical embodiments, the pancreatic neuroendocrine tumor is advanced and / or metastatic neuroendocrine tumor.

In some embodiments, the pancreatic neuroendocrine tumor is advanced and / or metastatic well differentiated neuroendocrine tumor.

In some embodiments, the pancreatic neuroendocrine tumor the one after failure of treatment with radiotherapy and / or chemotherapy.

In some embodiments, the neuroendocrine tumor is the one after failure of treatment with a molecular targeted drug (including but not limited to sunitinib or a salt thereof and / or everolimus). In some embodiments, the pancreatic neuroendocrine tumor is the one after failure of treatment with sunitinib or a salt thereof and / or everolimus.

In some embodiments, the neuroendocrine tumor is the one after failure of treatment with a somatostatin analog and / or an interferon- α and / or peptide receptor radionuclide.

Compound I can be administered in the free base form thereof, and can also be administered in the form of salts, hydrates and prodrugs thereof (the prodrugs will be converted into the free base form of Compound I in vivo). For example, a pharmaceutically acceptable salt of Compound I is within the scope of the present invention, and the salt can be produced from different organic acid and inorganic acid in accordance with well-known processes in the art.

In some embodiments, Compound I is administered in the form of hydrochloride thereof. In some embodiments, Compound I is administered in the form of monohydrochloride thereof. In some embodiments, Compound I is administered in the form of dihydrochloride thereof. In some embodiments, Compound I is administered in the crystalline form of hydrochloride thereof. In a certain embodiment, Compound I is administered in the crystalline form of dihydrochloride thereof. In some embodiments, Compound I is administered in the form of maleate thereof.

Compound I or a pharmaceutically acceptable salt thereof can be administered by a variety of routes, including, but not limited to the routes selected from the group consisting of: oral, parenteral, intraperitoneal, intravenous, intra-arterial, transdermal, sublingual, Intramuscular, rectal, transbuccal, intranasal, inhalation, vaginal, intraocular, topical, subcutaneous, intrafat, intra-articular, intraperitoneal or intrathecal. In a specific embodiment, it is administered orally.

The amount of Compound I or a pharmaceutically acceptable salt administered can be determined based on the severity of the disease, the response of the disease, any treatment-related toxicity, the age and health status of the patient. In some embodiments, the daily dose of Compound I or a pharmaceutically acceptable salt thereof is 3 mg to 30 mg. In some embodiments, the daily dose of Compound I or a pharmaceutically acceptable salt thereof is 5 mg to 20 mg. In some embodiments, the daily dose of Compound I or a pharmaceutically acceptable salt thereof is 8 mg to 16 mg. In some embodiments, the daily dose of Compound I or a pharmaceutically acceptable salt thereof is from 8 mg to 14 mg. In a specific embodiment, the daily dose of Compound I or a pharmaceutically acceptable salt thereof is 8 mg. In a specific embodiment, the daily dose of Compound I or a pharmaceutically acceptable salt thereof is 10 mg. In a specific embodiment, the daily dose of Compound I or a pharmaceutically acceptable salt thereof is 12 mg.

Compound I or the pharmaceutically acceptable salt thereof may be administered one or more times daily. In some embodiments, Compound I or the pharmaceutically acceptable salt thereof is administered once a day. In one embodiment, Compound I or the pharmaceutically acceptable salt thereof is administered once a day in the form of oral solid formulation.

The method of administration can be comprehensively determined according to the activity, toxicity, and tolerance of the patient and the like. Preferably, Compound I or the pharmaceutically acceptable salt thereof is administered in a manner of interval administration. The interval administration includes administration periods and withdrawal periods, and Compound I or the pharmaceutically acceptable salt thereof can be administered once or multiple times per day during the administration period. For example, Compound I or the pharmaceutically acceptable salt thereof is administered daily during the administration period, and then the administration is stopped for a period of time during the withdrawal period, followed by the administration period, and then the withdrawal period, which can be repeated multiple times. Wherein, the ratio of the administration period to the withdrawal period in days is 2: 0.5 to 5, preferably 2: 0.5 to 3, more preferably 2: 0.5 to 2, and even more preferably 2: 0.5 to 1.

In some embodiments, the administration is continuously performed for 2 weeks and then discontinued for 2 weeks. In some embodiments, the administration is continuously performed once daily for 14 days and then discontinued for 14 days, followed by continuously administration once daily for 14 days and then discontinued for 14 days, such an interval administration regimen with a two-week administration period and a two-week withdrawal period can be repeated many times.

In some embodiments, the administration is continuously performed for 2 weeks and then discontinued for 1 week. In some embodiments, the administration is continuously performed once daily for 14 days and then discontinued for 7 days, followed by continuously administration once daily for 14 days and then discontinued for 7 days, such an interval administration regimen with a two-week administration period and a one-week withdrawal period can be repeated many times.

In some embodiments, the administration is continuously performed for 5 days and then discontinued for 2 days. In some embodiments, the administration is continuously performed once daily for 5 days and then discontinued for 2 days, followed by continuously administration once daily for 5 days and then discontinued for 2 days, such an interval administration regimen with a five-day administration period and a two-day withdrawal period can be repeated many times.

In some specific embodiments, Compound I or the pharmaceutically acceptable salt thereof is administered orally at a dose of 12 mg once a day for 2 weeks and then withdrawn for 1 week.

In some specific embodiments, Compound I or the pharmaceutically acceptable salt thereof is administered orally at a dose of 10 mg once a day for 2 weeks and then withdrawn for 1 week.

In some specific embodiments, Compound I or the pharmaceutically acceptable salt thereof is administered orally at a dose of 8 mg once a day for 2 weeks and then withdrawn for 1 week.

In another aspect, the present application provides Compound I or the pharmaceutically acceptable salt thereof for use in the treatment of a neuroendocrine tumor, or for use in the preparation of a medicament for treating a neuroendocrine tumor.

In some typical embodiments, the neuroendocrine tumors includes, but is not limited to, carcinoid and pancreatic neuroendocrine tumor.

In some typical embodiments, the neuroendocrine tumor includes but is not limited to gastrointestinal neuroendocrine tumor, pulmonary neuroendocrine tumor, pancreatic neuroendocrine tumor, liver neuroendocrine tumor, prostate neuroendocrine tumor, bile duct neuroendocrine tumor, Breast neuroendocrine tumor, ampulla neuroendocrine tumor, thymus neuroendocrine tumor, mediastinal neuroendocrine tumor, retroperitoneal neuroendocrine tumor, thyroid neuroendocrine tumor, adrenal neuroendocrine tumor.

In some typical embodiments, the neuroendocrine tumor is a poorly differentiated neuroendocrine tumor. In some typical embodiments, the neuroendocrine tumor is a well differentiated neuroendocrine tumor.

In some typical embodiments, the neuroendocrine tumor is advanced and / or metastatic neuroendocrine tumor.

In some embodiments, the neuroendocrine tumor is advanced and / or metastatic well differentiated neuroendocrine tumor.

In some embodiments, the neuroendocrine tumor is the one after failure of treatment with radiotherapy and / or chemotherapy.

In some embodiments, the neuroendocrine tumor is the one after failure of treatment with a molecular targeted drug (including but not limited to sunitinib or a salt thereof and / or everolimus). In some embodiments, the neuroendocrine tumor is the one after failure of treatment with sunitinib or a salt thereof and / or everolimus.

In some embodiments, the neuroendocrine tumor is the one after failure of treatment with a somatostatin analog and / or an interferon- α and / or peptide receptor radionuclide.

In some embodiments, the pancreatic neuroendocrine tumor includes islet cell tumor, insulinoma, amine precursor uptake and decarboxylation cell tumors (APUDomas), vasoactive intestinal peptide tumor (VIP tumor), pancreatic peptide tumor, glucagon Tumor, and gastrin tumor.

In some typical embodiments, the pancreatic neuroendocrine tumor is a poorly differentiated neuroendocrine tumor. In some typical embodiments, the pancreatic neuroendocrine tumor is a well differentiated neuroendocrine tumor.

In some typical embodiments, the pancreatic neuroendocrine tumor is advanced and / or metastatic neuroendocrine tumor.

In some embodiments, the pancreatic neuroendocrine tumor is advanced and / or metastatic well differentiated neuroendocrine tumor.

In some embodiments, the pancreatic neuroendocrine tumor the one after failure of treatment with radiotherapy and / or chemotherapy.

In some embodiments, the neuroendocrine tumor is the one after failure of treatment with a molecular targeted drug (including but not limited to sunitinib or a salt thereof and / or everolimus). In some embodiments, the pancreatic neuroendocrine tumor is the one after failure of treatment with sunitinib or a salt thereof and / or everolimus.

In some embodiments, the neuroendocrine tumor is the one after failure of treatment with a somatostatin analog and / or an interferon- α and / or peptide receptor radionuclide.

Compound I can be administered in the free base form thereof, and can also be administered in the form of salts, hydrates and prodrugs thereof (the prodrugs will be converted into the free base form of Compound I in vivo). For example, a pharmaceutically acceptable salt of Compound I is within the scope of the present invention, and the salt can be produced from different organic acid and inorganic acid in accordance with well-known processes in the art.

In some embodiments, Compound I is administered in the form of hydrochloride thereof. In some embodiments, Compound I is administered in the form of monohydrochloride thereof. In some embodiments, Compound I is administered in the form of dihydrochloride thereof. In some embodiments, Compound I is administered in the crystalline form of hydrochloride thereof. In a certain embodiment, Compound I is administered in the crystalline form of dihydrochloride thereof. In some embodiments, Compound I is administered in the form of maleate thereof.

Compound I or the pharmaceutically acceptable salt thereof can be administered by a variety of routes, including, but not limited to the routes selected from the group consisting of: oral, parenteral, intraperitoneal, intravenous, intra-arterial, transdermal, sublingual, Intramuscular, rectal, transbuccal, intranasal, inhalation, vaginal, intraocular, topical, subcutaneous, intrafat, intra-articular, intraperitoneal or intrathecal. In a specific embodiment, it is administered orally.

The amount of Compound I or the pharmaceutically acceptable salt administered can be determined based on the severity of the disease, the response of the disease, any treatment-related toxicity, the age and health status of the patient. In some embodiments, the daily dose of Compound I or the pharmaceutically acceptable salt thereof is 3 mg to 30 mg. In some embodiments, the daily dose of Compound I or the pharmaceutically acceptable salt thereof is 5 mg to 20 mg. In some embodiments, the daily dose of Compound I or the pharmaceutically acceptable salt thereof is 8 mg to 16 mg. In some embodiments, the daily dose of Compound I or the pharmaceutically acceptable salt thereof is from 8 mg to 14 mg. In a specific embodiment, the daily dose of Compound I or the pharmaceutically acceptable salt thereof is 8 mg. In a specific embodiment, the daily dose of Compound I or the pharmaceutically acceptable salt thereof is 10 mg. In a specific embodiment, the daily dose of Compound I or the pharmaceutically acceptable salt thereof is 12 mg.

Compound I or the pharmaceutically acceptable salt thereof may be administered one or more times daily. In some embodiments, Compound I or the pharmaceutically acceptable salt thereof is administered once a day. In one embodiment, Compound I or the pharmaceutically acceptable salt thereof is administered once a day in the form of oral solid formulation .

The method of administration can be comprehensively determined according to the activity, toxicity, and tolerance of the patient and the like. Preferably, Compound I or the pharmaceutically acceptable salt thereof is administered in a manner of interval administration. The interval administration includes administration periods and withdrawal periods, and Compound I or the pharmaceutically acceptable salt thereof can be administered once or multiple times per day during the administration period. For example, Compound I or the pharmaceutically acceptable salt thereof is administered daily during the administration period, and then the administration is stopped for a period of time during the withdrawal period, followed by the administration period, and then the withdrawal period, which can be repeated multiple times. Wherein, the ratio of the administration period to the withdrawal period in days is 2: 0.5 to 5, preferably 2: 0.5 to 3, more preferably 2: 0.5 to 2, and even more preferably 2: 0.5 to 1.

In some embodiments, the administration is continuously performed for 2 weeks and then discontinued for 2 weeks. In some embodiments, the administration is continuously performed once daily for 14 days and then discontinued for 14 days, followed by continuously administration once daily for 14 days and then discontinued for 14 days, such an interval administration regimen with a two-week administration period and a two-week withdrawal period can be repeated many times.

In some embodiments, the administration is continuously performed for 2 weeks and then discontinued for 1 week. In some embodiments, the administration is continuously performed once daily for 14 days and then discontinued for 7 days, followed by continuously administration once daily for 14 days and then discontinued for 7 days, such an interval administration regimen with a two-week administration period and a one-week withdrawal period can be repeated many times.

In some embodiments, the administration is continuously performed for 5 days and then discontinued for 2 days. In some embodiments, the administration is continuously performed once daily for 5 days and then discontinued for 2 days, followed by continuously administration once daily for 5 days and then discontinued for 2 days, such an interval administration regimen with a five-day administration period and a two-day withdrawal period can be repeated many times.

In some specific embodiments, Compound I or the pharmaceutically acceptable salt thereof is administered orally at a dose of 12 mg once a day for 2 weeks and then withdrawn for 1 week.

In some specific embodiments, Compound I or the pharmaceutically acceptable salt thereof is administered orally at a dose of 10 mg once a day for 2 weeks and then withdrawn for 1 week.

In some specific embodiments, Compound I or the pharmaceutically acceptable salt thereof is administered orally at a dose of 8 mg once a day for 2 weeks and then withdrawn for 1 week.

In yet another aspect, the present invention provides a pharmaceutical composition for treating a neuroendocrine tumor, comprising Compound I or a pharmaceutically acceptable salt thereof and at least one pharmaceutically acceptable carrier.

The pharmaceutical composition may be the formulation suitable for oral, parenteral, intraperitoneal, intravenous, intra-arterial, transdermal, sublingual, intramuscular, rectal, transbuccal, intranasal, inhalation, vaginal, intraocular, topical, subcutaneous, intrafat, intra-articular, intraperitoneal and intrathecal administration; preferred formulations suitable for oral administration, including tablets, capsules, powders, granules, drip pills, pastes, powders, etc., Tablets and capsules are preferred. And the tablets may be conventional tablets, dispersible tablets, effervescent tablets, sustained-release tablets, controlled-release tablets, or enteric tablets, and the capsules may be conventional capsules, sustained-release capsules, controlled-release capsules, or enteric capsules. The oral formulation can be prepared by conventional methods using pharmaceutically acceptable carriers known in the art. Pharmaceutically acceptable carriers include fillers, absorbents, wetting agents, binders, disintegrants, lubricants, and the like. Fillers include starch, lactose, mannitol, microcrystalline cellulose, etc.; absorbents include calcium sulfate, calcium hydrogen phosphate, calcium carbonate, etc.; wetting agents include water, ethanol, etc.; binders include hypromellose, Povidone, microcrystalline cellulose, etc.; disintegrants include croscarmellose sodium, crospovidone, surfactants, low-substituted hydroxypropyl cellulose, etc.; lubricants include magnesium stearate, talc Powder, polyethylene glycol, sodium lauryl sulfate, colloidal silicon dioxide, talc, etc. Pharmaceutical excipients also include colorants, sweeteners, and the like.

In one embodiment, the pharmaceutical composition is a solid formulation suitable for oral administration. The composition may be in the form of a tablet or capsule, for example. In a specific embodiment, the pharmaceutical composition is a capsule. In a particular embodiment of the invention, the pharmaceutically acceptable carriers of the oral solid formulation include mannitol, microcrystalline cellulose, hydroxypropyl cellulose, magnesium stearate.

Compound I or the pharmaceutically acceptable salt thereof may be administered one or more times daily. In some embodiments, Compound I or the pharmaceutically acceptable salt thereof is administered once a day. In one embodiment, the oral solid formulation is administered once a day.

Preferably, the pharmaceutical composition is administered in a manner of interval administration. The interval administration includes administration periods and withdrawal periods, and Compound I or the pharmaceutically acceptable salt thereof can be administered one or more times per day during the administration period. For example, Compound I or the pharmaceutically acceptable salt thereof is administered daily during the administration period, and then the administration is stopped for a period of time during the withdrawal period, followed by the administration period, and then the withdrawal period, which can be repeated multiple times. Among them, the ratio of the administration period to the withdrawal period in days is 2: 0.5 to 5, preferably 2: 0.5 to 3, more preferably 2: 0.5 to 2, and even more preferably 2: 0.5 to 1.

In some embodiments, the administration is continuously performed for 2 weeks and then discontinued for 2 weeks. In some embodiments, the administration is continuously performed once daily for 14 days and then discontinued for 14 days, followed by continuously administration once daily for 14 days and then discontinued for 14 days, such an interval administration regimen with a two-week administration period and a two-week withdrawal period can be repeated many times.

In some embodiments, the administration is continuously performed for 2 weeks and then discontinued for 1 week. In some embodiments, the administration is continuously performed once daily for 14 days and then discontinued for 7 days, followed by continuously administration once daily for 14 days and then discontinued for 7 days, such an interval administration regimen with a two-week administration period and a one-week withdrawal period can be repeated many times.

In some embodiments, the administration is continuously performed for 5 days and then discontinued for 2 days. In some embodiments, the administration is continuously performed once daily for 5 days and then discontinued for 2 days, followed by continuously administration once daily for 5 days and then discontinued for 2 days, such an interval administration regimen with a five-day administration period and a two-day withdrawal period can be repeated many times.

In some specific embodiments, the pharmaceutical composition is administered orally at a dose of 12 mg once a day in a dosage regimen of two-week administration period and one-week withdrawal period.

In some specific embodiments, the pharmaceutical composition is administered orally at a dose of 10 mg once a day in a dosage regimen of two-week administration period and one-week withdrawal period.

In some specific embodiments, the pharmaceutical composition is administered orally at a dose of 8 mg once a day in a dosage regimen of two-week administration period and one-week withdrawal period.

In another aspect, the present application also provides a kit comprising (a) a pharmaceutical composition of at least one unit dose of Compound I or a pharmaceutically acceptable salt thereof, and (b) instructions for treating a neuroendocrine tumor. In some embodiments, a kit is provided, comprising (a) at least one unit dose of a formulation of Compound I or a pharmaceutically acceptable salt thereof suitable for oral administration, and (b) instructions for treating a neuroendocrine tumor with interval administration regimen . The "unit dose or unit dosage" refers to a pharmaceutical composition packaged in a single package for convenience of administration. For example, each tablet or capsule.

Herein, unless stated otherwise, the dosages and ranges provided herein are based on the molecular weight of the free base form of Compound I.

In the method, use, pharmaceutical composition or kit provided herein, Compound I or the pharmaceutically acceptable salt thereof can be separately administered to a patient as the sole active ingredient, or a second drug can also be included, Compound I or the pharmaceutically acceptable salt thereof, and the second drug can be administered to patients with neuroendocrine tumors simultaneously or sequentially. The second drug includes, but is not limited to, a somatostatin analogue, interferon- α, peptide receptor radionuclide, molecular targeted therapy, chemotherapy, and the like, such as anticancer drug (preferably antineuroendocrine tumor agent), anti-inflammatory and / or immunomodulatory and / or anti-allergic drug, the second drug also includes a drug for treating symptoms associated with a neuroendocrine tumor.

Herein, the crystalline form of the hydrochloride salt of compound I includes, but is not limited to, Form A, B, and C crystals disclosed in Chinese patent application CN102344438A, wherein the Form A and B crystals are substantially free of crystal water and other solvents. Form C crystal is a crystal containing two crystalline waters. In some embodiments, the crystalline form of the dihydrochloride salt of Compound I is Form A crystal.

Unless otherwise stated, for the purposes of this application, the following terms used in this specification and claims shall have the following meanings.

"Patient" refers to human.

"Pharmaceutically acceptable" refers to suitable for the preparation of a pharmaceutical composition, which are generally safe, non-toxic, and neither biologically or otherwise undesirable, and also acceptable for use in human medicine.

"Pharmaceutically acceptable salt" includes, but is not limited to, an acid addition salt with inorganic acid such as hydrochloric acid, hydrobromic acid, sulfuric acid, nitric acid, phosphoric acid, and the like; or with organic acid such as acetic acid, trifluoroacetic acid, and propionic acid , Hexanoic acid, heptanoic acid, cyclopentanepropionic acid, glycolic acid, pyruvate, lactic acid, malonic acid, succinic acid, malic acid, maleic acid, fumaric acid, tartaric acid, citric acid, benzoic acid, cinnamic acid, mandelic acid, methanesulfonic acid, ethanesulfonic acid, 1,2-ethanedisulfonic acid, 2-hydroxyethanesulfonic acid, benzenesulfonic acid, p-chlorobenzenesulfonic acid, p-toluenesulfonic acid, 3-phenylpropionic acid, trimethylacetic acid , tert-butylacetic acid, dodecylsulfate, gluconic acid, glutamic acid, hydroxynaphthoic acid, salicylic acid, stearic acid, and the like.

"Therapeutically effective amount" refers to an amount of the compound that is sufficient to control of the disease when administered to human for treating a disease.

"Treatment or treat" refers to any administration of a therapeutically effective amount of a compound, and includes:
(1) inhibiting the disease in a human who is experiencing or showing the pathology or symptomology of the disease (i.e., preventing further development of the pathology and / or symptomology), or
(2) ameliorating the disease in a human who is experiencing or showing the pathology or symptomology of the disease (i.e., reversing the pathology and / or symptomology).

"PR" is abbreviation of "partial remission", which refers to the sum of the diameter of target lesions of tumor is reduced by more than 30% from the baseline level.

"PD" is abbreviation of "disease progression", which refers to the sum of the diameters of target lesions of tumor increased by more than 20% from the baseline level.

"SD" is abbreviation of "stable disease", which refers to the sum of the diameter of target lesions of tumor is neither decreased to the the level of PR nor increased to the level of PD, i.e., between PR and PD.

"PFS" refers to progression-free survival.

"OS" refers to overall survival.

"po" refers to oral.

"qd" refers to once a day.

"Bid" refers to twice a day.

### EXAMPLES

The following embodiments are to illustrate to the technical solution of the present application, and the protection scope of the present application is not limited to the scope of the embodiments.

### Example 1 1-[[[[4-(4-fluoro-2-methyl-1H-indol-5-yl)oxy-6-methoxyquinolin-7-yl]oxy]methyl] Cyclopropylamine dihydrochloride

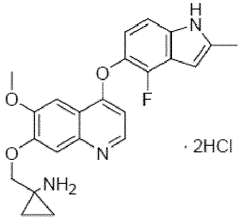

According to the method of Example 24 in WO2008112407, 1-[[[4-(4-fluoro-2-methyl-1H-indole-5-yl)oxy-6-methoxyquinolin-7-yl]oxy]methyl]cyclopropylamine was synthesized, and then referring to the preparation method of "Examples of Salt Formation" in the specification of WO2008112407, the title compound was prepared.

Alternatively, the title compound can be prepared by referring to the method disclosed in Chinese patent application CN102344438A.

### Example 2 Preparation of Capsules of 1-[[[4-(4-fluoro-2-methyl-1H-indol-5-yl)oxy-6-methoxy quinolin-7-yl] oxy]methyl]cyclopropylamine dihydrochloride (Dihydrochloride of Compound I)

| | |
|---|---|
| Ingredient/excipient | amount (1000 capsules) |
| Dihydrochloride of Compound I | 14.16 g(equivalent to 12 g Compound I) |
| Mannitol | 89 g |
| Microcrystalline cellulose | 138.4 g |
| Hydroxypropyl cellulose | 5.9 g |
| Magnesium stearate | 0.99 g |

Dihydrochloride of Compound I was grinded and sifted with a 80 mesh sieve, and then mixed uniformly with mannitol and hydroxypropyl cellulose; the prescribed amount of microcrystalline cellulose was subsequently added, mixed uniformly and sifted with a 0.8mm sieve; and finally, the prescribed amount of magnesium stearate was added and mixed uniformly, and the obtained mixture was filled into capsules.

The capsule with different amount of dihydrochloride of Compound I can be prepared by reference to the above proportion and formulation.

### Example 3

A 69-year-old male patient was diagnosed with ampulla neuroendocrine tumor(tumor in liver caudate were suspected of metastases) after excision of liver masses and gallbladder, and biliary enterostomy by postoperative pathology, MRI(Magnetic Resonance Imaging) showed enlarged uncinate process of the head of pancreas with nodular-like changes, and low biliary obstruction, abdominal CT showed that a soft tissue shadow of approximately 3.2 × 2.4 cm in the uncinate area of the head of pancreas, nodules in left inner lobe of the liver which was suspected of a hemangioma, and the right anterior lobe nodule of the liver. One month after the operation, local radiotherapy was performed for the masses in the head of pancreas, which was 30Gy / 3Gy / 10f (3gy for each radiation, 30gy for 10 radiotherapy sessions), evaluated as SD after the treatment. EP regimen (gemcitabine + cisplatin) was given for two cycles from the fourth to sixth month after the operation, during which IV ° myelosuppression occurred. Abdominal MRI showed that the liver metastases were larger than before and the disease progressed two years after operation. A CT scan showed that the total diameter of the target lesions (liver metastases) was 25 mm. Then the patient was enrolled in the clinical trial and was treated once a day with a daily dose of 12 mg of dihydrochloride of Compound I capsules (two-week administration period and one-week withdrawal period as one treatment cycle).

During the subsequent treatment of dihydrochloride of Compound I capsules, CT scan was performed. The imaging examination after two treatment cycles showed that the sum of the diameter of the metastases decreased to 21 mm; then decreased to 16 mm after four treatment cycles, and the therapy efficacy was evaluated as PR; decreased to 15mm after six treatment cycles, and the therapy efficacy was evaluated as PR; then still 15mm after eight treatment cycles, and evaluated as PR; followed by a dose adjustment to 10mg after eleven cycles as II ° fatigue occurred; the sum of the diameter was 15mm after twelve treatment cycles , and evaluated as PR; and was 15mm after sixteen treatment cycles, evaluated as PR; then further decreased to 13mm after twenty two treatment cycles and evaluated as PR.

Overall, the treatment was well tolerated, during the treatment of dihydrochloride of Compound I capsules, the main side effect was II ° fatigue, II ° decreased PLT, I ° diarrhea, and I ° hand-foot skin reaction.

### Example 4

A 34-year-old male patient was diagnosed with neuroendocrine tumor which tend to be atypical carcinoid after a chest tumor puncture by pathology exam, then the patient was treated with chemotherapy of paclitaxel, etoposide and cisplatin for three cycles, a CT scan was performed after the treatment, efficacy of the therapy was evaluated as SD, followed by Right anterior mediastinal mass resection and partial right upper lobe resection. Postoperative pathology showed that neuroendocrine carcinoma infiltrated in thymic tissue, vascular tumor thrombus was visible, the tumor was 12 × 9 × 5.5cm, and tumor invaded the visceral wall, while without invasion of lung tissue, pericardial fat, metastasis was not found in lymph gland. Local chest radiotherapy was performed in the first to second month after operation, and evaluated as SD after the treatment, during which III° radiation esophagitis occurred. Then EP regimen (gemcitabine + cisplatin) was given for 2 cycles, follow-up examination every three months showed a stable disease and no recurrence. Then CT scan showed the disease progressed, and multiple metastases were found in bilateral pleura, bilateral kidney, left adrenal gland, and lymph nodes which was performed in the twenty third month after operation. Temozolomide and capecitabine was then given for 2 cycles from the 23^{rd} month to 25th month after operation, and efficacy was evaluated as SD (large), during this period, pain in both lower extremities occurred, and even got worse. Then 4 mg of zoledronic acid was given once a month, the efficacy was evaluated as PD by CT scan after two cycles of the treatment, followingly, Shanlong (octreotide acetate microspheres for injection) was given for 1 cycle, the efficacy was evaluated as PD by CT scan which was performed after 27 months postoperatively, and the sum of the target lesion diameters was 179 mm. The patient was then enrolled in a clinical trial and was treated once a day with a daily dose of 12 mg of dihydrochloride of Compound I capsules (two-week administration period and one-week withdrawal period as one treatment cycle).

During the subsequent treatment of dihydrochloride of Compound I capsules, CT scan was performed periodically. The imaging examination after two treatment cycles showed that the sum of the diameter of the target lesions decreased to 151 mm; then decreased to 135 mm after four treatment cycles, then decreased to 127mm after six treatment cycles, and then decreased to 115mm after eight treatment cycles, the therapy efficacy was evaluated as PR; then decreased to 106mm after ten treatment cycles, the efficacy was evaluated as PR; and the sum of the diameter of the target lesions was 119mm after 13 treatment cycles (evaluated as PR), 120 mm after 16 treatment cycles.

Overall, the treatment was well tolerated, during the treatment of dihydrochloride of Compound I capsules, the main side effect was III ° ALT elevation, II ° proteinuria, I ° hypertriglyceridemia.

### Example 5

A 65-year-old female patient was diagnosed with retroperitoneal neuroendocrine tumor by biopsy, then the patient was treated with Saisheng (Bozhi glycopeptide injection) and thymosin , while the reexamination showed no response, then she was treated with sunitinib malate, and the efficacy was evaluated as PD.

Thereafter, the patient was treated with a daily dose of 12 mg of dihydrochloride of Compound I capsules once a day (two-week administration period and one-week withdrawal period as one treatment cycle).

CT scan showed that the sum of the diameters of the target lesions (retroperitoneal lymph node metastasis, together with lung and liver metastases) was 35 mm before the treatment of dihydrochloride of Compound I. During the subsequent treatment of dihydrochloride of Compound I capsules, CT scan was performed periodically. The imaging examination after two treatment cycles showed that the sum of the diameter of the target lesions decreased to 24 mm (efficacy was evaluated as PR); then decreased to 22 mm after four treatment cycles, then decreased to 21mm after six treatment cycles(efficacy was evaluated as PR), and was still 21mm after eight and fourteen treatment cycles(efficacy was evaluated as PR), then decreased to 20mm after seventeen treatment cycles, and also was 20mm after 21 treatment cycles(efficacy was evaluated as PR), then decreased to 19mm after 25 treatment cycles (efficacy was evaluated as PR), 16mm after 31 treatment cycles (efficacy was evaluated as PR), and was 19mm after 37 treatment cycles (efficacy was evaluated as PR).

Overall, the treatment was well tolerated, during the treatment of dihydrochloride of Compound I capsules, the main side effect was II ° decreased WBC, I ° hypothyroidism, I °decreased neutrophils, and I ° diarrhea.

### Example 6

A 40-year-old male patient was diagnosed with pancreatic neuroendocrine tumor by pathology combined with history and immunohistochemistry, epithelial cell nests were visible in liver tissue(liver metastasis). Laparotomy and resection of pancreatic body, tail and spleen was performed under general anesthesia, ShanLong (octreotide acetate microspheres for injection) and traditional Chinese medicine were given one month after operation, and the efficacy was evaluated as PD six months after operation. Then the patient was enrolled into the clinical trial and was treated with a daily dose of 12 mg of dihydrochloride of Compound I capsules once a day (two-week administration period and one-week withdrawal period as one treatment cycle).

CT scan showed that the sum of the diameters of the target lesions (right adrenal metastases, liver metastases) was 59 mm before the treatment of dihydrochloride of Compound I. During the subsequent treatment of dihydrochloride of Compound I capsules, CT scan was performed. The imaging examination after two treatment cycles showed that the sum of the diameter of the target lesions decreased to 41 mm (efficacy was evaluated as PR); then decreased to 37 mm after four treatment cycles(efficacy was evaluated as PR), then decreased to 35mm after six treatment cycles(efficacy was evaluated as PR), and then decreased to 32mm after eight treatment cycles, and was 34mm after 14 treatment cycles(efficacy was evaluated as PR), then decreased to 32mm after 17 treatment cycles (efficacy was evaluated as PR), and was 35mm after 21 treatment cycles (efficacy was evaluated as PR).

Overall, the treatment was well tolerated, during the treatment of dihydrochloride of Compound I capsules, the main side effect was I° diarrhea, I ° hand-foot skin reaction, II °ALT elevation, II° bilirubin elevation, I°proteinuria.

### Example 7

Patients diagnosed with neuroendocrine tumors, after oral administration of 12 mg of dihydrochloride of Compound I capsules once a day (two-week administration period and one-week withdrawal period as one treatment cycle), C0 * denoted the sum of the diameters of the target lesions before the treatment (unit: mm), and C2*, C4*, C6*, C8*, C10*, C14* denoted the sum of the diameters of the target lesions after 2, 4, 6, 8, 10, 14 treatment cycles respectively (unit: mm), the results were shown in Table 1.

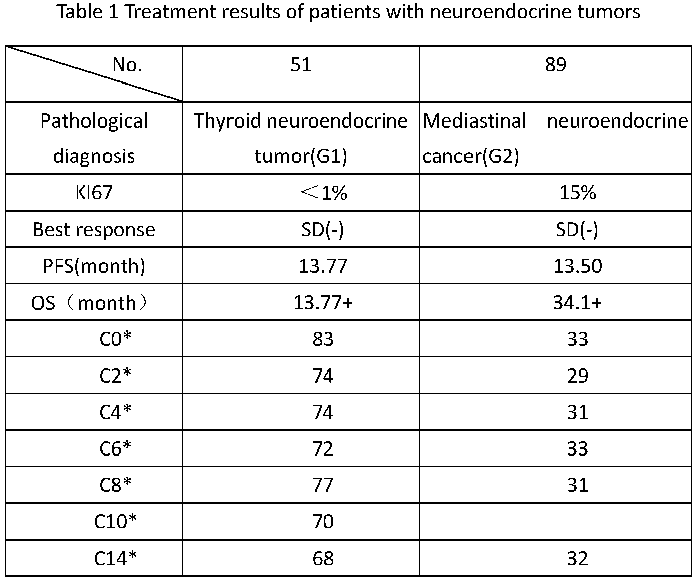

### Example 8

A 60-year-old male patient, whose postoperative immunohistochemistry result was HER2 (0), CK (+), CgA (+), Syn (+), and Ki-67 (+60%) after laparoscopic assisted radical gastrectomy which was performed on March 31, 2016, was diagnosed with ulceratively poorly differentiated neuroendocrine carcinoma (G3, about 90%) and mixed poorly differentiated adenocarcinoma (about 10%) in lesser curvature of stomach. After oral administration of tegafur for eight cycles, a tumor marker CEA was 3004ng / ml on December 26, 2016, and an enhanced CT showed space occupying lesions of the second hepatic hilum and pancreatic head suspected of lymph nodes metastasis and fusion; and multiple ring enhancing lesions in the liver suspected of liver metastasis. IP regimen (Irinotecan 200mg, d1, 100mg, d8; cisplatin 30mg, d1-3) was given on January 04, and February 10, 2017, respectively. The CEA was 824.400ng/ml on February 09, 2017 , and 5621.000ng/ml on August 23, 2017. Systemic chemotherapy with EOS regimen (EPI (epirubicin) 80 mg d1, LOHP (oxaliplatin) 100 mg d1, and S-1 (tegafur capsules) 50 mg PO bid 1-14; Q3W) was given for two cycles from August 25, 2017 with significantly relieved pain in upper abdomen, and efficacy was evaluated as PR, then two more cycles of the treatment was given, the 5th to 7th cycles of chemotherapy treatment were given on December 13, 2017, January 12, 2018, and February 06, 2018, respectively. CT scan on April 19, 2018 showed that the sum of the diameters of target lesions was 18.2 mm (lymph nodes of porta hepatis, pancreatic head and retroperitoneum), then the patient was enrolled into the clinical trial of dihydrochloride of Compound I capsules.

A daily dose of 12 mg of dihydrochloride of Compound I capsule was given once a day from April 21, 2018 (two-week administration period and one-week withdrawal period, i.e. three weeks as one treatment cycle). After two treatment cycles, CT scan showed the sum of the diameters of the metastatic lesions (lymph nodes of porta hepatis, pancreatic head and retroperitoneum) decreased to 18.1mm on May 29, 2018, then decreased to 18.0mm (lymph nodes of porta hepatis, pancreatic head and retroperitoneum) on July 12, 2018 after four treatment cycles, the efficacy was evaluated as SD. As of September 2018, the patient was still in treatment.

Overall, the treatment was well tolerated, during the treatment of dihydrochloride of Compound I capsules, the main side effect was II° hypertension, I ° Glycosuria, I °elevated bilirubin, I° weight loss, and I °appetite loss.

### Example 9

A 55-year-old male patient, who underwent gastric biopsy on April 15, 2017, the pathology suggested poorly differentiated cancer, and immunohistochemical results were: CK (+), CDX2 (small focal weak +), CK7 (-), CK (+), Syn (+), CgA (+), TTP-1 (partial +), CD56 (+), Ki-67 (80%), was diagnosed as neuroendocrine cancer(poorly differentiated) on April 24, 2017.

Chemotherapy with Irinotecan (100 mg d1, d8, d15) and carboplatin (600 mg d1), Q3W was given from April 26, 2017 which was well tolerated, then the regimen was adjusted to 270mg of Irinotecan (d1) and 700 mg of carboplatin (d1) for three treatment cycles. A total gastrectomy was performed on August 1, 2017, the postoperative pathology suggested ulceratively poorly differentiated adeno(neuro)endocrine carcinoma (approximately 20% of adenocarcinoma and approximately 80% of neuroendocrine carcinoma, Lauren's diffuse type) in lesser curvature of stomach. The EP regimen (VP-16 (etoposide) 150 mg d1, 200 mg, d2-3; DPP (cisplatin) 40 mg, d1-3; Q3W) was given from November 14, 2017 for 2 cycles, and the efficacy was evaluated as PD, then PLOT regimen (150 mg of oxaliplatin, d1; 150 mg of liposome-paclitaxel, d1; 300 mg of calcium folinate, d1; 3.6 g of tegafur, CIV (intravenous infusion) for 24 hours) was given from December 29, 2017 for one treatment cycle. The FLOT regimen (150 mg of oxaliplatin, d1; 150 mg of liposome-paclitaxel, d1; 300 mg of calcium folinate, d1; 3.6 g of tegafur, CIV for 24 hours) and endostar (210mg civ 120h) was given on January 11, 2018, January 24, 2018, February 11, 2018, February 27, 2018, and March 13, 2018 respectively, the efficacy was evaluated as PD after the chemotherapy. Then the chemotherapy was adjusted to CAP regimen (cyclophosphamide 800mg d1; cisplatin 40mg d1-3; and doxorubicin liposomal 40mg d1) from April 10, 2018, CT scan showed the sum of diameter of the target lesions was 47.8mm (S5 and S6 of the liver) on May 30, 2018, then the patient diagnosed as gastric neuroendocrine carcinoma with postoperative liver metastasis was enrolled into the clinical trial.

A daily dose of 12 mg of dihydrochloride of Compound I capsule was given once a day from June 2, 2018 (two-week administration period and one-week withdrawal period, i.e. three weeks as one treatment cycle). After two treatment cycles, CT scan showed the sum of the diameters of the metastatic lesions (S5 and S6 of the liver) was decreased to 45.9mm on July 11, 2018, the efficacy was evaluated as SD. As of September 2018, the patient was still in treatment. Overall, the treatment was well tolerated, during the treatment of dihydrochloride of Compound I capsules, the main side effect was I° hypertension, I ° Glycosuria, I °elevated bilirubin.

### Example 10

A 67-year-old male patient, underwent a laparotomy on August 18, 2016, the gallbladder, distal part of the stomach, duodenum, and part of the pancreas were removed. Postoperative pathology displayed that a mass was visible on the section from the common bile duct to the major duodenal papilla , 2.3^{∗}0.8^{∗}1.5cm, and immunohistochemistry was Ki-67 (30%). The patient was diagnosed with ampullary neuroendocrine tumor (high proliferative activity, mitotic count, 13/0HPF) on November 4, 2016, which was located in the ampulla, the common bile duct, duodenal papilla and major pancreatic duct were invaded, intravascular tumor thrombus was visible and pancreatic tissue was also invaded, immunohistochemistry: AFP (-).

Chemotherapy with tegafur (orally, 60 mg) and temozolomide (200 mg) was given from November 18, 2016 for nine treatment cycles, and the efficacy was evaluated as SD, maintenance therapy with tegafur was then given from August 8^{th}, 2017 to February 2018, during this period, the treatment was discontinued for one cycle for elevated bilirubin, CT suggested the disease progressed. 10mg of everolimus (qd) was administered orally from February 7 to May 25, 2018, and the efficacy was evaluated as SD on March 29, 2018. CT and MRI were performed on May 25, 2018, the results suggested multiple metastatic tumors in the liver, and the disease progressed.

A CT scan was performed on July 2, 2018, which showed that the total diameter of the target lesions was 44 mm (space occupying lesions of the right lobe of liver), then the patient was enrolled into the clinical trial of dihydrochloride of Compound I capsules. A daily dose of 12 mg of dihydrochloride of Compound I capsule was given once a day from July 4, 2018 (two-week administration period and one-week withdrawal period, i.e. three weeks as one treatment cycle). After two treatment cycles, CT scan showed the sum of the diameters of the metastatic lesions (space occupying lesions of the right lobe of liver) decreased to 35mm on August 15, 2018, the efficacy was evaluated as SD. As of September 2018, the patient was still in treatment, during which the treatment was generally well tolerated without adverse events..

### Example 11

A 45-year-old male patient was diagnosed with neuroendocrine tumor with liver metastases(G2) after irregular resection of the right liver, proximal subtotal gastrectomy and splenectomysurgery on July 13, 2016, postoperative pathology report showed that poorly differentiated neuroendocrine tumor remained in the greater curvature of the stomach with small focal necrosis, which was classified as mild-moderate atypia, and mitosis was visible easily, the disease was diagnosed as neuroendocrine tumor. One cycle of treatment with 40 mg of cisplatin and 100 mg of VP-16 (etoposide) was given from September 6, 2016, then radiofrequency ablation and puncture biopsy of liver metastases were performed on April 20, 2017, postoperative pathology report showed tumor infiltration in the liver tissue, and immunohistochemical results showed Ki-67 (+ 30%), liver magnetic resonance imaging showed disease progression on June 26, 2017. The chemotherapy with 280 mg of irinotecan, 1500 mg of capecitabine and 100mg of thalidomide was given for 3 cycles from July 6, 2017 to August 23, 2017. A CT scan which was performed on June 7, 2018, showed significant enlargement of metastases in the right lobe of liver, occupying almost the entire right liver, and the disease progressed.

A CT scan which was performed on July 4, 2018, and the result showed that the sum of the diameters of the target lesions was 180 mm (huge space occupying lesions of liver), then the patient was enrolled into the clinical trial of dihydrochloride of Compound I capsules. A daily dose of 12 mg of dihydrochloride of Compound I capsule was given once a day from July 6, 2018 (two-week administration period and one-week withdrawal period, i.e. three weeks as one treatment cycle). After two treatment cycles, CT scan showed the sum of the diameters of the metastatic lesions (huge space occupying lesions of liver) decreased to 150mm on August 14, 2018, the efficacy was evaluated as SD. As of September 2018, the patient was still in treatment, during which the treatment was generally well tolerated without adverse events.

## Claims

1. Use of Compound I or a pharmaceutically acceptable salt thereof in the manufacture of a medicament for treating a neuroendocrine tumor,

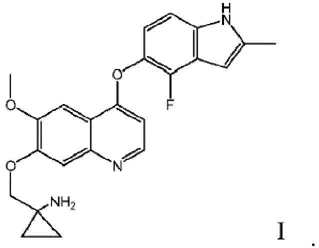

2. The use according to claim 1, wherein the neuroendocrine tumor is gastrointestinal neuroendocrine tumors, pulmonary neuroendocrine tumor, pancreatic neuroendocrine tumor, liver neuroendocrine tumor, prostate neuroendocrine tumor, bile duct neuroendocrine tumor, breast neuroendocrine tumor, ampulla neuroendocrine tumor, thymus neuroendocrine tumor, mediastinal neuroendocrine tumor, retroperitoneal neuroendocrine tumor, thyroid neuroendocrine tumor or adrenal neuroendocrine tumor; the pancreatic neuroendocrine tumor is preferably islet cell tumor, insulinoma, amine precursor uptake and decarboxylioma, vasoactive intestinal peptide tumor, glucagon tumor, pancreatic peptide tumor, and gastrinoma.

3. The use according to claim 1 or 2, wherein the neuroendocrine tumor is a poorly differentiated neuroendocrine tumor or a well differentiated neuroendocrine tumor.

4. The use according to any one of claims 1-3, wherein the neuroendocrine tumor is advanced and / or metastatic neuroendocrine tumor.

5. The use according to any one of claims 1-4, wherein the neuroendocrine tumor is the one after failure of treatment with radiotherapy and / or chemotherapy and / or molecular targeted therapy.

6. The use according to any one of claims 1-5, wherein the pharmaceutically acceptable salt is a salt formed by Compound I and any of the following acids: hydrochloric acid, hydrobromic acid, sulfuric acid, nitric acid, phosphoric acid, acetic acid, trifluoroacetic acid, propionic acid, hexanoic acid, heptanoic acid, cyclopentanepropionic acid, glycolic acid, pyruvate, lactic acid, malonic acid, succinic acid, malic acid, maleic acid, fumaric acid, tartaric acid, citric acid , benzoic acid, cinnamic acid, mandelic acid, methanesulfonic acid, ethanesulfonic acid, 1,2-ethanedisulfonic acid, 2-hydroxyethanesulfonic acid, benzenesulfonic acid, p-chlorobenzenesulfonic acid, p-toluenesulfonic acid, 3-phenylpropionic acid, trimethyl acetic acid, tert-butyl acetic acid, dodecylsulfate, gluconic acid, glutamic acid, hydroxynaphthoic acid, salicylic acid, stearic acid; preferably hydrochloride or maleate, more preferably dihydrochloride.

7. The use according to any one of claims 1-6, wherein the medicament is a formaulation suitable for oral, parenteral, intraperitoneal, intravenous, intraarterial, transdermal, sublingual, intramuscular, rectal, transbuccal, intranasal, inhalable, vaginal, intraocular, topical, subcutaneous, intrafat, intra-articular, intraperitoneal or intrathecal administration; preferably a formulation suitable for oral administration; more preferably tablets, capsules, powders, granules, drip pills, pastes or pulvis, more preferably tablets or capsules.

8. The use according to any one of claims 1 to 7, **characterized in that** the daily dose of compound I or the pharmaceutically acceptable salt thereof is 3 mg to 30 mg, preferably 5 mg to 20 mg, more preferably 8 mg to 16 mg, still more preferably 8 mg to 14 mg, and most preferably 8 mg, 10 mg, or 12 mg.

9. The use according to any one of claims 1 to 8, **characterized in that** compound I or the pharmaceutically acceptable salt thereof is administered in a manner of interval administration which includes administration periods and withdrawal periods, preferably the ratio of the administration period to the withdrawal period in days is 2: 0.5 to 5, more preferably 2: 0.5 to 3, more preferably 2: 0.5 to 2, and even more preferably 2: 0.5 to 1; the more preferable interval administration regimen is selected from one of the following: two-week administration period and two-week withdrawl period, two-week administration period and one-week withdrawl period, five-day administration period and two-day withdrawl period, and the interval administration regimen can be repeated many times .

10. A method for treating a neuroendocrine tumor, wherein the method comprises administering a therapeutically effective amount of Compound I or a pharmaceutically acceptable salt thereof to a patient in need of treatment,
